(19) Europäisches Patentamt
European Patent Office
Office européen des brevets

(11) Veröffentlichungsnummer: **0 139 627 B1**

# (12) EUROPÄISCHE PATENTSCHRIFT

(45) Veröffentlichungstag der Patentschrift: **22.05.91**

(51) Int. Cl.[5]: **C07D 243/38**, C07D 405/12, //A61K31/55

(21) Anmeldenummer: **84890185.6**

(22) Anmeldetag: **09.10.84**

Die Akte enthält technische Angaben, die nach dem Eingang der Anmeldung eingereicht wurden und die nicht in dieser Patentschrift enthalten sind.

## (54) Verfahren zur Herstellung von neuen in 5-Stellung substituierten 5,10-Dihydro-11H-dibenzo(b,e)(1,4)diazepin-11-onen.

(30) Priorität: **19.10.83 DD 255766**

(43) Veröffentlichungstag der Anmeldung:
**02.05.85 Patentblatt 85/18**

(45) Bekanntmachung des Hinweises auf die Patenterteilung:
**22.05.91 Patentblatt 91/21**

(84) Benannte Vertragsstaaten:
**AT CH DE FR GB LI**

(56) Entgegenhaltungen:
**EP-A- 0 044 989**
**DE-A- 2 022 790**
**DE-B- 1 795 183**
**FR-A- 2 016 008**

**CHEMICAL ABSTRACTS, Band 66, Nr. 1, 2. Januar 1967, Seite 134, Nr. 1384z, Columbus, Ohio, US; L. TOLDY et al.: "Derivatives of piperazine. I. Derivatives of 3,4,5-trimethoxybenzoylpiperazine, a new group of compounds with antiulcerogenic activity" & ACTA CHIM. ACAD. SCI. HUNG. 49(3), 265-285(1966)**

(73) Patentinhaber: **VEB Arzneimittelwerk Dresden**
**Wilhelm-Pieck-Strasse 35**
**O-8122 Radebeul 1(DE)**

(72) Erfinder: **Rüger, Carla, Dr.**
**Zillerstrasse 7**
**O-8122 Radebeul(DE)**
Erfinder: **Röhnert, Helmut, Dr.**
**Weinbergstrasse 33**
**O-8122 Radebeul(DE)**
Erfinder: **Bahr, Fritz, Dr.**
**Permoserstrasse 3/002**
**O-8019 Dresden(DE)**
Erfinder: **Lohmann, Dieter, Dr.**
**Hoflössnitzstrasse 30**
**O-8122 Radebeul(DE)**
Erfinder: **Hoffmann, Evelyn, Dipl.Biol.**
**Ernst-Thälmann-Strasse 33**
**O-8122 Radebeul(DE)**
Erfinder: **Bartsch, Reni, Dr.**
**Stephensonstrasse 11**
**O-8045 Dresden(DE)**
Erfinder: **Schumann, Steffen, Dr.**
**Löscherstrasse 33**
**O-8019 Dresden(DE)**

**EP 0 139 627 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

(74) Vertreter: **Puchberger, Rolf, Dipl. Ing. et al**
**Patentanwälte, Dipl. Ing. Georg Puchberger Dipl. Ing. Rolf Puchberger Dipl. Ing. Peter Puchberger Singerstrasse 13 Postfach 55 A-1010 Wien(AT)**

## Beschreibung

Anwendungsgebiet der Erfindung

Die Erfindung betrifft ein Verfahren zur Herstellung von neuen in 5-Stellung substituierten 5,10-Dihydro-11H-dibenzo-[b,e][1,4]diazepin-11-onen der allgemeinen Formel I und ihren physiologisch verträglichen Salzen mit anorganischen oder organischen Säuren, worin $R^1$ Wasserstoff oder Chlor, $R^2$ Wasserstoff oder Methyl, $R^3$ Wasserstoff, Methyl oder 2-Hydroxyäthyl, $R^4$ 2-Hydroxyethyl, N-Morpholylcarbonylmethyl oder 2-Acetylaminoäthyl oder auch $R^3$ und $R^4$ zusammen mit dem N-Atom, an das sie gebunden sind, einen Piperazinorest, der an der Iminogruppe durch einen 2-Furan-carbonyl- oder 3,4,5-Trimethoxybenzoylrest substituiert ist, und A eine Gruppe der Formel $-CH_2-$; $-CH_2-CH_2-$ oder $-CH(CH_3)-$ oder eine direkte Bindung bedeuten kann.

Die Verbindungen der Formel I besitzen eine ausgezeichnete antiulzeröse und sekretionshemmende Wirkung.

Charakteristik der bekannten technischen Lösungen

Es sind in 5-Stellung substitutierte 11H-Dibenzo[b,e][1,4]diazepin-11-one (DE-AS 2 022 790, 2 065 570, 1 936 670, 1 795 176, 1 931 487; EP 44 989; DE-OS 3 028 001) und in 11-Stellung substituierte 5,6-Dihydro-11H-pyrido[2,3-b]-[1,4]benzodiazepin-6-one (z.B. Pirenzepin) (DD-PS 135 490) bekannt, die eine gute antiulzeröse und sekretionshemmende Wirkung aufweisen, wobei geringe oder keine Nebenwirkungen (z.B. mydriatische Wirkung) im therapeutischen Dosisbereich auftreten.

Ziel der Erfindung

Durch die Erfindung ist es möglich, neue in 5-Stellung substituierte 5,10-Dihydro-11H-dibenzo[b,e][1,4]-diazepin-11-one der Formel I sowie deren physiologisch verträgliche Salze mit anorganischen oder organischen Säuren mit wertvollen pharmakologischen Eigenschaften herzustellen.

Wie aus den nachfolgenden Ausführungen ersichtlich, wirken sie insbesondere ulkus- und sekretionshemmend.

So wurden zum Beispiel die Wirkungen der Verbindungen

-8-Chlor-5,10-dihydro-5-[bis-(2-hydroxyäthyl)aminoacetyl]-11H-dibenzo[b,e] [1,4]diazepin-11-on = A

-5,10-Dihydro-5-[bis-(2-hydroxyäthyl)-aminoacetyl]-11H-dibenzo[b,e][1,4]diazepin-11-on = B

-5,10-Dihydro-5-(2-{4-[(2-furanyl)-carbonyl]-1-piperazinyl}-acetyl)-11H-dibenzo[b,e] [1,4]diazepin-11-on = C

-8-Chlor-5,10-dihydro-5-(2- { 4-[(2-furanyl)-carbonyl]-1-piperazinyl}-acetyl)-10-methyl-11H-dibenzo[b,e][1,4]-diazepin-11-on = D

-5,10-Dihydro-10-methyl-5-[bis-(2-hydroxyäthyl)-aminoacetyl]- 11H-dibenzo[b,e] [1,4]diazepin-11-on = E

an den Modellen Immobilisationsulkus und Phenylbutazonulkus überprüft, die mydriatische Wirkung sowie die akute Toxizität ermittelt und mit den Standardverbindungen Pirenzepin und Atropin verglichen.

Die Hemmwirkung auf die Bildung von streßinduzierten Magenulzera wurde nach der Methode von D.A.Brodie, Gastroenterolody, 38 (1960), 50, an weiblichen Ratten im Gewicht von 150 - 180 Gramm vom

VEB Versuchstierproduktion Schönwalde bestimmt. Die Nahrungskarenz vor Versuchsbeginn betrug 48 Stunden, bei Wasser ad libitum. Die Testsubstanzen wurden unmittelbar vor Versuchsbeginn oral, mittels Schlundsonde appliziert. Die Immobilisation erfolgte mittels Gipsbinden über 18 Stunden. Während der Versuchsdauer war die Raumtemperatur konstant 20° C. Nach Versuchsende wurden die Tiere durch Äther getötet, die Mägen entnommen, entlang der kleinen Kurvatur aufgeschnitten und die Drüsenschleimhautdefekte nach Zahl und Länge im Vergleich zur Kontrollgruppe beurteilt. Für die statistische Berechnung diente die Probit-Regressions-Analyse.

Die Hemmwirkung der Verbindungen auf phenylbutazoninduzierte Magenulzera wurde in Anlehnung an das Modell von Beattie und Mitarbeiter, Arzneimittelforschung, 29, (1979), 1812 ermittelt. Für die Untersuchung kamen weibliche Ratten der hauseigenen Zucht im Gewicht von 150 - 180 Gramm zur Anwendung.

Die Nahrungskarenz vor Versuchsbeginn betrug 24 Stunden, bei Wasser ad libitum. Während des Versuchs wurden die Tiere im Klimaraum bei einer Temperatur zwischen 22° C und 23° C gehalten. Die Ratten erhielten zwei Tage hintereinander jeweils 7 Uhr morgens eine s.c. Injektion von 150 mg/kg Phenylbutazon und die Tötung erfolgte sechs Stunden nach der letzten Injektion durch Nackenschlag. Die herauspräparierten Mägen wurden entlang der kleinen Kurvatur aufgeschnitten und die Schleimhautdefekte nach Zahl und Länge beurteilt. Die Applikation der Prüfsubstanzen erfolgte oral, eine halbe Stunde vor jeder Phenylbutazoninjektion.

Die Wirkung der Substanzen auf die Größe der Pupille wurde an weiblichen Mäusen im Gewicht zwischen 18 und 23 Gramm nach der Versuchsordnung von R.Pulewka, Exp.Pathol. Pharmakol. 167, (1932), 307 untersucht. 30, 60, 90, 120, 180 und 240 Minuten nach oraler Präparatgabe wurde die Pupillenweite bestimmt und die x-fache Veränderung zum Ausgangswert berechnet. Als $ED_3$-fach gilt die Dosis, bei der eine dreifache Pupillenerweiterung im Vergleich zum Ausgangswert auftritt.

Die akute Toxizität wurde an jeweils 5 männlichen und 5 weiblichen Mäusen im Gewicht von 18 - 23 Gramm pro dosi bestimmt. Die Applikation der Prüfsubstanzen erfolgte oral. Die Berechnung der $LD_{50}$ wurde nach Litchfield und Wilcoxon durchgeführt.

Die Ergebnisse aller Untersuchungen sind in folgender Tabelle zusammengefaßt:

| Substanz | Immobilisationsulkus $ED_{50}$(mg/kg) | Phenylbutazonulkus $ED_{50}$(mg/kg) | Pupillomotorik $ED_{3}$-fach (mg/kg) | akute Toxizität $LD_{50}$(mg/kg) | Quotient Mydriasis $ED_{3}$-fach / Ulkushemmung $ED_{50}$ (Immobilisationsulkus) |
|---|---|---|---|---|---|
| A | 6 | 5 | 30 | 2000 | 5 |
| B | 1 | 1,6 | 4 | 2000 | 4 |
| C | 14 | 20 | 67 | 2000 | 5 |
| D | 26 | 36 | 250 | 1600 | 10 |
| E | 27 | 22 | 95 | 1000 | 3,5 |
| Pirenzepin | 11 | 18 | 30 | 1460 | 3 |
| Atropin | 0,6 | 0,8 | 0,8 | 850 | 1 |

Die Ergebnisse zeigen, daß die neuen Verbindungen die Entstehung von Magenulzera unterschiedlicher Genese verhindern. An beiden Ulkusmodellen zeigen sie eine gleichmäßige Wirkungsstärke, die mit der strukturell ähnlichen Verbindung Pirenzepin vergleichbar bzw. besser ist. Für alle Verbindungen ist das Verhältnis $ED_{3\text{-fach}}$ der Pupillomotorik zur $ED_{50}$ Ulkushemmung größer bzw. wesentlich größer als für Atropin. Damit treten im therapeutischen Dosisbereich keine mydriatischen Wirkungen auf.

Darlegung des Wesens der Erfindung

Der vorliegenden Erfindung liegt die Aufgabe zugrunde, neue in 5-Stellung substituierte 5,11-Dihydro-11H-dibenzo[b,e]-[1,4]diazepin-11-one der allgemeinen Formel I sowie deren physiologisch verträgliche Salze mit anorganischen und organischen Säuren und wertvollen pharmakologischen Eigenschaften herzustellen.

Entsprechend der vorliegenden Erfindung können die Verbindungen der allgemeinen Formel I auf verschiedene Weise hergestellt werden.

So kann man entweder

a) ein 5,10-Dihydro-5-halogenacyl-11H-dibenzo[b,e][1,4]diazepin-11-on der allgemeinen Formel II, worin $R^1$, $R^2$ und A die oben genannte Bedeutung besitzen und X ein Halogenatom bedeutet, mit einem Amin der allgemeinen Formel III, in der $R^3$ und $R^4$ die oben genannte Bedeutung haben, umsetzen.

Die Umsetzung kann bei Temperaturen zwischen Raumtemperatur und 150 °C, vorzugsweise zwischen 50 und 120 °C und in einem indifferenten organischen Lösungsmittel, gegebenenfalls in Gegenwart eines säurebindenden Mittels wie einem tertiären Amin z.B. Triäthylamin, Pyridin, Dimethylanilin, einem Alkalicarbonat, Alkalihydrogencarbonat, Natriumacetat oder einem Überschuß der Verbindung der allgemeinen Formel III, durchgeführt werden.

Bei Gegenwart eines säurebindenden Mittels kann die Verbindung der allgemeinen Formel III auch als Hydrochlorid eingesetzt werden.

Als indifferente organische Lösungsmittel können Alkohole wie Äthanol, n-Propanol, Isopropanol, Äther wie Dioxan, Tetrahydrofuran, Ketone wie Aceton, Cyclohexanon, aromatische Kohlenwasserstoffe wie Benzen, Toluen, halogenierte aliphatische oder aromatische Kohlenwasserstoffe wie Chloroform, 1,2-Dichloräthan, Chlorbenzen oder ein Überschuß der umzusetzenden Verbindung der allgemeinen Formel III verwendet werden.

Die Ausgangsverbindungen der allgemeinen Formel II sind teilweise literaturbekannt oder lassen sich in Anlehnung an literaturbekannte Methoden (A.M. Monro et al., J. Med.Chem. 6, 255 (1963)) durch Umsetzung eines 5,10-Dihydro-11H-dibenzo[b,e][1,4]diazepin-11-ons der allgemeinen Formel IV, worin $R^1$ und $R^2$ die obige Bedeutung besitzen, mit einem Halogenacylhalogenid der allgemeinen Formel V, worin A die oben genannte Bedeutung besitzt, und X und X', die gleich oder verschieden sein können, Halogenatome wie Chlor oder Brom bedeuten, gegebenenfalls in Gegenwart halogenwasserstoffbindender Mittel, herstellen.

Diese Umsetzung erfolgt vorzugsweise in einem inerten organischen Lösungsmittel bei erhöhten Temperaturen. Als Lösungsmittel können aromatische Kohlenwasserstoffe wie Benzen, Toluen, Xylen, cyclische Äther wie Dioxan, Tetrahydrofuran, aber auch Ester wie Äthansäureäthylester verwendet werden.

Bei der Herstellung der Verbindungen der Formel II können als halogenwasserstoffbindende Mittel anorganische Basen wie Alkalicarbonate oder Alkalihydrogencarbonate eingesetzt werden oder, wenn A die Methylengruppe oder eine direkte Bindung ist, auch tertiäre organische Amine wie Triäthylamin, N,N-Dimethylanilin oder. Pyridin verwendet werden.

Die erhaltenen Halogenacylprodukte der allgemeinen Formel II können ohne weitere Reinigung in die erfindungsgemäße Umsetzung eingesetzt werden.

Man kann aber auch

b) eine Verbindung der allgemeinen Formel VI, worin $R^1$ und $R^2$ die oben genannte Bedeutung besitzen, in einem indifferenten organischen Lösungsmittel, zum Beispiel Alkoholen wie Äthanol, n-Propanol, Isopropanol, Ketonen wie Aceton, hochsiedenden Äthern wie Tetrahydrofuran, Dioxan oder aromatischen Kohlenwasserstoffen wie Benzen oder Toluen, mit einem Amin der allgemeinen Formel III, worin $R^3$ und $R^4$ die oben genannte Bedeutung besitzen, unter Erwärmen, vorzugsweise bis zur Siedetemperatur des Reaktionsgemisches, zu den entsprechenden Verbindungen der allgemeinen Formel I umsetzen, in denen die Reste $R^1$ bis $R^4$ die oben genannte Bedeutung besitzen und A eine Äthylengruppe ist.

Die als Ausgangsverbindungen eingesetzten Verbindungen der allgemeinen Formel VI sind teilweise bekannt und können nach an sich bekannten Methoden hergestellt werden, indem man beispielsweise eine Verbindung der allgemeinen Formel IV, worin $R^1$ und $R^2$ die oben genannte Bedeutung besitzen, mit einem Acrylsäurehalogenid umsetzt oder indem man eine Verbindung der allgemeinen Formel II, worin $R^1$, $R^2$ und X die oben genannte Bedeutung besitzen und A eine Äthylengruppe ist, in einem inerten organischen Lösungsmittel, zum Beispiel höher siedenden Äthern wie Tetrahydrofuran und Dioxan oder aromatischen Kohlenwasserstoffen wie Benzen oder Toluen, in Gegenwart tertiärer organischer Amine wie Triäthylamin, Pyridin oder N,N-Dimethylanilin, vorzugsweise bis zur Siedetemperatur des Reaktionsgemisches erhitzt. Die gleiche Eliminierungsreaktion zu den 5-Acryloylverbindungen der allgemeinen

Formel VI läuft auch als Nebenreaktion bei der Herstellung der beim ersten Verfahren als Ausgangsstoffe verwendeten Verbindungen der allgemeinen Formel II ab (A = Äthylen), wenn man dort Verbindungen der allgemeinen Formel IV mit Halogenacylhalogeniden der allgemeinen Formel V (A = Äthylen) unter Zusatz von tertiären Basen als halogenwasserstoff bindenden Mitteln umsetzt. Die dann in den Ausgangsverbindungen der allgemeinen Formel II (A = Äthylen) als Nebenprodukte enthaltenen Verbindungen der allgemeinen Formel VI stören die weitere Umsetzung nicht nur nicht, sondern nehmen selbst an der Herstellung der Verbindungen der allgemeinen Formel I (A = Äthylen) teil.

Man kann aber auch

c) eine Verbindung der allgemeinen Formel IV, worin $R^1$ und $R^2$ die oben genannte Bedeutung besitzen, mit einer Verbindung der allgemeinen Formel VII, in der $R^3$, $R^4$ und A die oben genannte Bedeutung besitzen und Y eine Hydroxylgruppe oder ein Halogenatom, bzw. wenn A eine direkte Bindung ist, nur ein Halogenatom bedeutet, oder deren Salze in einem inerten organischen Lösungsmittel, zum Beispiel halogenierten Kohlenwasserstoffen wie Chloroform oder Dichlormethan, gegebenenfalls unter Erwärmen, vorzugsweise in der Siedehitze, umsetzen.

Wird die Umsetzung mit einer Verbindung der allgemeinen Formel VII durchgeführt, in der Y eine Hydroxylgruppe ist, wird vorzugsweise in Gegenwart von 1-Methyl-2-halogen-pyridiniumjodid und Triäthylamin (1:1) gearbeitet.

Die erhaltenen Verbindungen der allgemeinen Formel I können nach bekannten Methoden mit anorganischen oder organischen Säuren in ihre physiologisch verträglichen Salze überführt werden.

Als Säuren können zum Beispiel Chlorwasserstoffsäure, Bromwasserstoffsäure, Schwefelsäure, Phosphorsäure, Weinsäure, Zitronensäure, Fumarsäure, Maleinsäure, Bernsteinsäure oder Oxalsäure Verwendung finden.

## Ausführungsbeispiele

### Beispiel 1

8-Chlor-5,10-dihydro-5-[bis-(2-hydroxyäthyl)-aminoacetyl]-11H-dibenzo[b,e][1,4]diazepin-11-on

6,4 g (0,02 Mol) 8-Chlor-5-chloracetyl-5,10-dihydro-11H-dibenzo[b,e][1,4]diazepin-11-on werden in 200 ml Chloroform zum Sieden erhitzt und 8,4 g (0,08 Mol) Diäthanolamin zugetropft.

Das Reaktionsgemisch rührt man 2 Stunden am Rückfluß, engt im Vakuum ein, kocht den Rückstand mit Butylacetat aus und kristallisiert aus Acetonitril unter Zusatz von CFO-Kohle um. Die Ausbeute beträgt 3,4 g (43,7 % der Theorie), Schmelzpunkt: 188 - 192° C (unter Zersetzung).

| Analyse für $C_{19}H_{20}N_3O_4Cl$: | berechnet | gefunden |
|---|---|---|
| C: | 58,54 % | 58,32 % |
| H: | 5,17 % | 4,85 % |
| N: | 10,78 % | 10,69 % |
| Cl: | 9,09 % | 9,18 % |

Hydrochlorid

7,8 g (0,02 Mol) 8-Chlor-5,10-dihydro-5-[bis-(2-hydroxyäthyl)-aminoacetyl]-11H-dibenzo[b,e][1,4]-diazepin-11-on werden in 80 ml Isopropanol in der Hitze gelöst und noch warm mit 40 ml ca. 13%-iger isopropanolischer Salzsäure versetzt. Nach dem Abkühlen wird das kristalline Produkt abgesaugt und aus 140 ml 93%-igem Äthanol umkristallisiert. Die Ausbeute beträgt 4,6 g (53,8 % der Theorie), Schmelzpunkt: 225 - 226° C (unter Zersetzung).

| Analyse für $C_{19}H_{21}N_3O_4Cl_2$: | berechnet | gefunden |
|---|---|---|
| C: | 53,53 % | 53,79 % |
| H: | 4,97 % | 4,79 % |
| N: | 9,86 % | 9,83 % |
| Cl: | 16,63 % | 16,34 % |

Das als Ausgangsprodukt eingesetzte 8-Chlor-5-chloracetyl-5,10-dihydro-11H-dibenzo[b,e][1,4]diazepin-11-on kann wie folgt hergestellt werden:

39,2 g (0,16 Mol) 8-Chlor-5,10-dihydro-11H-dibenzo[b,e][1,4]diazepin-11-on werden in 400 ml Xylen suspendiert, auf dem Ölbad auf 100° C Badtemperatur erwärmt, mit 18,1 g (0,16 Mol) Chloracetylchlorid versetzt und 3 Stunden bei der angegebenen Badtemperatur gerührt. Anschließend tropft man nochmals 9,04 g (0,08 Mol) Chloracetylchlorid zu, rührt eine Stunde bei 100° C Badtemperatur, saugt nach dem Abkühlen das Kristallisat ab und trocknet bei Raumtemperatur. Das Rohprodukt wird aus 800 ml Acetonitril unter Zusatz von EPN-Kohle umkristallisiert.
Die Ausbeute beträgt 34,1 g (66,3 % der Theorie).
Schmelzpunkt: 245,5° C - 247,5° C (unter Zersetzung).

In analoger Weise wurden durch Umsetzung der entsprechenden Verbindungen der allgemeinen Formel IV die folgenden Verbindungen der allgemeinen Formel II erhalten:

- mit Chloracetylchlorid in Xylen als Lösungsmittel das 5-Chloracetyl-5,10-dihydro-11H-dibenzo[b,e]-[1,4]diazepin-11-on, F. 238,5 - 140° C (aus Butylacetat)
- mit Chloracetylchlorid in Toluen als Lösungsmittel das 8-Chlor-5-chloracetyl-5,10-dihydro-10-methyl-11H-dibenzo[b,e][1,4]diazepin-11-on, F. 220 - 221,5° C,
- mit Chloracetylchlorid in Toluen als Lösungsmittel das 5-Chloracetyl-5,10-dihydro-10-methyl-11H-dibenzo[b,e][1,4]diazepin-11-on, F. 178,5 - 181° C (aus Acetonitril),
- mit 3-Chlorpropionylchlorid in Toluen als Lösungsmittel das 8-Chlor-5-(3-chlorpropionyl)-5,10-dihydro-11H-dibenzo[b,e][1,4]diazepin-11-on, F. 223 - 225° C,
- mit 3-Chlorpropionylchlorid in Toluen als Lösungsmittel das 8-Chlor-5-(3-chlorpropionyl)-5,10-dihydro-10-methyl-11H-dibenzo[b,e][1,4]diazepin-11-on, F. 163,5 - 166° C,
- mit 3-Chlorpropionylchlorid in Toluen als Lösungsmittel das 5-(3-Chlorpropionyl)-5,10-dihydro-10-methyl-11H-dibenzo[b,e][1,4]diazepin-11-on, F. 181,5 - 182,5° C,
- mit 2-Brompropionylchlorid in Toluen als Lösungsmittel das 5-[(2-Brom)-propionyl]-8-chlor-5,10-dihydro-11H-dibenzo[b,e][1,4]diazepin-11-on, F. 268 - 269,5° C und
- mit 2-Chlorpropionylchlorid in Toluen als Lösungsmittel das 5-[(2-Chlor)-propionyl]-8-chlor-5,10-dihydro-11H-dibenzo[b,e][1,4]diazepin-11-on, F. 281 - 284° C (unter Zersetzung).

In analoger Weise, jedoch bei Raumtemperatur, wurden folgende Verbindungen hergestellt:

- mit Phosgenüberschuß in Äthansäureäthylester oder Dioxan als Lösungsmittel das 8-Chlor-5-chlorcarbonyl-5,10-dihydro-11H-dibenzo[b,e][1,4]diazepin-11-on, F. 259 - 262° C,
- mit Phosgenüberschuß in Tetrahydrofuran als Lösungsmittel das 5-Chlorcarbonyl-5,10-dihydro-11H-dibenzo[b,e][1,4]diazepin-11-on, F. 251 - 254° C (unter Zersetzung) (aus Aceton).

<u>Beispiel 2</u>

8-Chlor-5,10-dihydro-5-(2-{4[(2-furanyl)-carbonyl]-1-piperazinyl}-acetyl)-11H-dibenzo[b,e][1,4]diazepin-11-on

12,8 g (0,04 Mol) 8-Chlor-5-chloracetyl-5,10-dihydro-11H-dibenzo[b,e][1,4]diazepin-11-on werden in 200 ml Toluol mit 15,4 g (0,08 Mol) 1-[(2-Furanyl)-carbonyl]-piperazin 6 Stunden am Rückfluß gerührt. Zum Reaktionsgemisch gibt man nach dem Abkühlen 200 ml Wasser, rührt gut durch und saugt das Gemisch ab. Der Filterrückstand wird mit Wasser gewaschen, in 100 ml Chloroform aufgenommen, dasselbe mit 100 ml Wasser ausgeschüttelt und anschließend im Vakuum eingeengt.

Den Rückstand versetzt man zweimal mit Chloroform und engt jedesmal im Vakuum ein,dann gibt man 20 ml Isopropanol zu, erhitzt 10 Minuten auf dem Dampfbad, saugt das Kristallisat nach dem Animpfen ab und kristallisiert aus 200 ml Isopropanol unter Zusatz von CFO-Kohle um. Die Ausbeute beträgt 12,6 g (67,8 % der Theorie), Schmelzpunkt: 190 - 193° C.

| Analyse für $C_{24}H_{21}N_4O_4Cl$: | berechnet | gefunden |
|---|---|---|
| C: | 62,00 % | 62,68 % |
| H: | 4,55 % | 4,74 % |
| N: | 12,05 % | 11,42 % |
| Cl: | 7,63 % | 7,68 % |

Hydrochlorid

22 g (0,047 Mol) 8-Chlor-5,10-dihydro-5-(2-{4[(2-furanyl)-carbonyl]-1-piperazinyl}-acetyl)-11H-dibenzo-[b,e][1,4]diazepin-11-on werden in 350 ml Isopropanol unter leichtem Erwärmen gelöst und nach vorsichtigem Abkühlen 200 ml ca. 19 %-ige isopropanolische Salzsäure zugegeben. Nach einstündigem Stehen engt man die Lösung im Vakuum zur Trockne ein, gibt nochmals Isopropanol zu, engt wieder ein und kristallisiert den etwas schmierigen Rückstand aus 100 ml n-Propanol mit CFO-Kohle um. Anschließend wird nochmals aus 50 %-igem Äthanol umkristallisiert. Die Ausbeute beträgt 15,2 g (61,8 % der Theorie), Schmelzpunkt: 209 - 215° C (unter Zersetzung).

Analyse für das Hydrat $C_{24}H_{24}N_4O_5Cl_2$:

| | berechnet | gefunden |
|---|---|---|
| C: | 55,50 % | 54,99 % |
| H: | 4,66 % | 4,93 % |
| N: | 10,79 % | 11,37 % |
| Cl: | 13,65 % | 13,70 % |

<u>Beispiel 3</u>

8-Chlor-5,10-dihydro-5-{[(N-morpholylcarbonylmethyl)amino]-acetyl}-11H-dibenzo[b,e][1,4]diazepin-11-on

6,4 g (0,02 Mol) 8-Chlor-5-chloracetyl-5,10-dihydro-11H-dibenzo[b,e][1,4]diazepin-11-on werden in 100 ml Dichloräthan mit 14,4 g (0,08 Mol) Glycylmorpholid und 10,0 g (1,00 Mol) Triäthylamin 3 Stunden am Rückfluß gerührt. Bei dieser Umsetzung wird eine gewisse Menge disubstituiertes Nebenprodukt gebildet. Das Reaktionsgemisch schüttelt man dreimal mit 50 ml Wasser aus, anschließend zweimal mit 10 ml 1:1 verdünnter HCl und stellt dann die vereinigten salzsauren Phasen mit 1:1 verdünnter Ammoniaklösung alkalisch. Die alkalisch gestellte Lösung wird dreimal mit 50 ml Chloroform, die vereinigten Chloroformphasen einmal mit 10 ml Wasser ausgeschüttelt, anschließend trocknet man über $Na_2SO_4$ und engt im Vakuum zur Trockne ein.

Hydrochlorid

Den obigen Rückstand (10,2 g) löst man in 100 ml trockenem Aceton, leitet unter Rühren bei Raumtemperatur HCl-Gas bis zur sauren Reaktion ein, läßt noch 1/2 Stunde nachrühren, saugt das Kristallisat ab, wäscht mit wenig trockenem Aceton nach und kristallisiert nach dem Trocknen aus 96 %-igem Äthanol mit CFO-Kohle um. Die Ausbeute beträgt 3,1 g (32,1 % der Theorie), Schmelzpunkt: 212° C (unter Zersetzung).

Analyse für das Hydrat $C_{21}H_{24}N_4O_5Cl_2$:

| | berechnet | gefunden |
|---|---|---|
| C: | 52,18 % | 51,76 % |
| H: | 4,79 % | 4,94 % |
| N: | 11,59 % | 11,47 % |
| Cl: | 14,66 % | 14,68 % |
| $H_2O$: | 3,73 % | 3,10 % |

Beispiel 4

8-Chlor-5,10-dihydro-5-{[4-(3,4,5-trimethoxybenzoyl)-1-piperazinyl]-acetyl}-11H-dibenzo[b,e][1,4]-diazepin-11-on

6,4 g (0,02 Mol) 8-Chlor-5-chloracetyl-5,10-dihydro-11H-dibenzo[b,e][1,4]diazepin-11-on werden in 50 ml Toluen mit 8,37 g (0,025 Mol) N-(3,4,5-Trimethoxybenzoyl)-piperazin und 2,53 g (0,025 Mol) Triäthylamin 2 Stunden am Rückfluß gerührt.

Das Reaktionsgemisch engt man danach zur Trockne ein, setzt 80 ml Chloroform zu,erwärmt bis fast alles Festprodukt in Lösung gegangen ist, schüttelt die Chlorformphase zweimal mit 50 ml Wasser aus, wobei eine klare Lösung entsteht und extrahiert anschließend zweimal mit 20 ml 2:1 verdünnter Salzsäure. Die vereinigten salzsauren Phasen werden mit 20 ml Chloroform ausgeschüttelt, mit 1:1 verdünnter Ammoniaklösung alkalisch gestellt und dreimal mit 50 ml Chloroform extrahiert.

Die Chloroformphase schüttelt man einmal mit 20 ml Wasser aus, trocknet sie über $Na_2SO_4$ und engt zur Trockne ein. Der Rückstand wird mit 20 ml Acetonitril aufgekocht und nach dem Abkühlen das Kristallisat abgesaugt.

Nach dem Trocknen gibt man es in 50 ml Isopropanol, erwärmt und setzt in der Hitze isopropanolische Salzsäure bis zur sauren Reaktion zu, wobei das gesamte Produkt in Lösung geht. Die Lösung wird mit CFO-Kohle gekocht, filtriert und zur Trockne eingeengt.

Den Rückstand kocht man nochmals mit Isopropanol auf und saugt nach dem Erkalten das Hydrochlorid ab. Die Ausbeute beträgt 5,24 g (41,7 % der Theorie), Schmelzpunkt: 236 °C (unter Zersetzung).

Analyse für das Hydrat $C_{29}H_{33}N_4O_{7,5}Cl$:

| | berechnet | gefunden |
|---|---|---|
| C: | 55,42 % | 55,07 % |
| H: | 5,29 % | 5,33 % |
| N: | 8,91 % | 8,21 % |
| Cl: | 11,45 % | 11,20 % |
| $H_2O$:(1,5 Mol) | 4,40 % | 4,50 % |

Beispiel 5

5,10-Dihydro-5-[bis-(2-hydroxyäthyl)-aminoacetyl]-11H-dibenzo[b,e][1,4]diazepin-11-on

5,73 g (0,02 Mol) 5-Chloracetyl-5,10-dihydro-11H-dibenzo[b,e][1,4]diazepin-11-on und 100 ml Chloroform werden zum Sieden erhitzt, innerhalb 1 Stunde 8,44 g (0,08 Mol) Diäthanolamin in wenig Chloroform zugetropft und 3 Stunden bei Siedetemperatur gerührt. Nach dem Abkühlen schüttelt man die Chloroformp-

hase mit 20 ml $NaHCO_3$-Lösung (2 g $NaHCO_3$ auf 50 ml Wasser), zweimal mit 10 ml und einmal mit 5 ml 1:2 verdünnter Salzsäure aus. Die vereinigten salzsauren Phasen werden in 50 ml 1:1 verdünnte Ammoniaklösung eingegossen. Die basische Lösung extrahiert man anschließend dreimal mit 50 ml Chloroform, trocknet die vereinigten Chloroformphasen über $Na_2SO_4$ und engt zur Trockne ein. Der Rückstand wird mit 10 ml Isopropanol versetzt, unter Erwärmen in Lösung gebracht und nach dem Animpfen das Kritallisat abgesaugt. Man kristallisiert aus 35 ml Acetonitril um. Die Ausbeute beträgt 1,8 g (25,3 % der Theorie), Schmelzpunkt: 144° C (unter Zersetzung).

| Analyse für $C_{19}H_{21}N_3O_4$: | berechnet | gefunden |
|---|---|---|
| C: | 64,21 % | 64,65 % |
| H: | 5,96 % | 6,12 % |
| N: | 11,82 % | 11,82 % |

Hydrochlorid

7 g (0,015 Mol) 5,10-Dihydro-5-[bis-(2-hydroxyäthyl)-aminacetyl]-11H-dibenzo[b,e][1,4]diazepin-11-on werden in 210 ml Isopropanol unter Erwärmen gelöst. Nach dem Abkühlen gibt man unter Rühren 7 ml isopropanolische Salzsäure (ca. 19 %-ig) und anschließend 35 ml Äther zu, läßt das Reaktionsgemisch 12 Stunden stehen, saugt das Kristallisat ab und trocknet es im Exsikkator. Das Rohprodukt wird aus 60 ml Methanol mit CFO-Kohle umkristallisiert. Die Ausbeute beträgt 2,99 g (39,5 % der Theorie), Schmelzpunkt: 211 - 214° C (unter Zersetzung).

| Analyse für $C_{19}H_{22}N_3O_4Cl$: | berechnet | gefunden |
|---|---|---|
| C: | 58,24 % | 58,23 % |
| H: | 5,66 % | 5,73 % |
| N: | 10,72 % | 11,01 % |
| Cl: | 9,05 % | 9,15 % |

Beispiel 6

5,10-Dihydro-5-(2-{4-[(2-furanyl)-carbonyl]-1-piperazinyl}-acetyl)-11H-dibenzo[b,e][1,4]diazepin-11-on

5,8 g (0,02 Mol) 5-Chloracetyl-5,10-dihydro-11H-dibenzo[b,e][1,4]diazepin-11-on, 100 ml Chloroform und 7,2 g (0,04 Mol) 1-[(2-Furanyl)-carbonyl]-piperazin werden 7 Stunden unter Rühren am Rückfluß erhitzt. Nach der Umsetzung schüttelt man die erkaltete Reaktionslösung zweimal mit 30 ml Wasser, zweimal mit 10 ml und einmal mit 5 ml 1:1 verdünnter Salzsäure aus. Die vereinigten salzsauren Phasen werden einmal mit 30 ml Chloroform ausgeschüttelt und in 100 ml 1:1 verdünnte Ammoniaklösung eingegossen, wobei ein öliges Produkt ausfällt, das man nach dem Abgießen der wäßrig-alkalischen Phase in 50 ml Chloroform aufnimmt. Die wäßrig-alkalische Phase wird noch zweimal mit 50 ml Chloroform ausgeschüttelt und die vereinigten Chloroformphasen werden nach dem Trocknen über $Na_2SO_4$ im Vakuum zur Trockne eingeengt. Den Rückstand kocht man mit 20 ml Toluol auf, saugt das Kristallisat noch dem Abkühlen ab und kristallisiert aus 110 ml 96 %-igem Äthanol mit CFO-Kohle um. Die Ausbeute beträgt 3,8 g (44,2 % der Theorie), Schmelzpunkt: 230 - 233° C.

| Analyse für $C_{24}H_{22}N_4O_4$: | berechnet | gefunden |
|---|---|---|
| C: | 66,97 % | 66,54 % |
| H: | 5,15 % | 5,19 % |
| N: | 13,02 % | 12,33 % |

Hydrochlorid

10 g (0,023 Mol) 5,10-Dihydro-5-(2-{4-[(2-furanyl)-carbonyl]-1-piperazinyl}-acetyl)-11H-dibenzo[b,e][1,4]-diazepin-11-on werden in 675 ml Isopropanol gelöst, mit CFO-Kohle 15 Minuten gekocht, blank gesaugt und noch heiß mit 10 ml isopropanolischer Salzsäure (ca. 19 %-ig) versetzt. Beim Abkühlen kristallisiert das Hydrochlorid mit einem Mol Wasser.

Analyse für das Hydrat $C_{24}H_{25}N_4O_5Cl$:

| | berechnet | gefunden |
|---|---|---|
| C: | 59,44 % | 59,08 % |
| H: | 5,20 % | 5,38 % |
| N: | 11,55 % | 11,03 % |
| Cl: | 7,31 % | 7,17 % |
| $H_2O$: | 3,70 % | 3,40 % |

Beispiel 7

5,10-Dihydro-5-[bis-(2-hydroxyäthyl)-aminoacetyl]-10-methyl-11H-dibenzo[b,e][1,4]diazepin-11-on

6,02 g (0,02 Mol) 5-Chloracetyl-5,10-dihydro-10-methyl-11H-dibenzo[b,e][1,4]diazepin-11-on werden in 100 ml 1,2-Dichloräthan zum Rückfluß erhitzt, 8,44 g (0,08 Mol) Diäthanolamin langsam zugetropft und das Reaktionsgemisch 4 Stunden am Rückfluß gerührt. Nach dem Abkühlen schüttelt man einmal mit 30 ml 10 %-iger $NaHCO_3$-Lösung, zweimal mit 10 ml und einmal mit 5 ml 1:1 verdünnter Salzsäure und die vereinigten salzsauren Phasen einmal mit 30 ml Chloroform aus.

Zu den salzsauren Phasen werden 100 ml 1:1 verdünnte Ammoniaklösung gegeben, dreimal mit 30 ml Chloroform ausgeschüttelt, die Chloroformphasen über $Na_2SO_4$ getrocknet und im Vakuum zur Trockne eingeengt.

Den Rückstand löst man in 30 ml trockenem Aceton und leitet Chlorwasserstoff bis zur sauren Reaktion ein. Dabei fällt ein schmieriges Produkt an, das beim Aufkochen kristallisiert.Das Hydrochlorid wird aus 40 ml 96 %-igem Äthanol mit CFO-Kohle umkristallisiert und bei 100° C und 1 Torr getrocknet. Die Ausbeute beträgt 4,0 g (49,3 % der Theorie), Schmelzpunkt: 196- 199° C (unter Zersetzung).

| Analyse für $C_{20}H_{24}N_3O_4Cl$: | berechnet | gefunden |
|---|---|---|
| C: | 59,19% | 59,33 % |
| H: | 5,96 % | 5,97 % |
| N: | 10,35 % | 10,42 % |
| Cl: | 8,74 % | 8,70 % |

Beispiel 8

8-Chlor-5,10-dihydro-5-[bis-(2-hydroxyäthyl)-aminoacetyl]-10-methyl-11H-dibenzo[b,e][1,4]diazepin-11-on

6,7 g (0,02 Mol) 8-Chlor-5-chloracetyl-5,10-dihydro-10-methyl-11H-dibenzo[b,e][1,4]diazepin-11-on und 100 ml Chloroform werden unter Rühren zum Sieden erhitzt und 8,44 g (0,08 Mol) Diäthanolamin in 5 ml Chloroform langsam zugetropft. Man rührt 7 Stunden am Rückfluß, schüttelt das erkaltete Reaktionsgemisch zweimal mit 50 ml Wasser aus, extrahiert die Chloroformphase zweimal mit 10 ml und einmal mit 5 ml 1:1 verdünnter Salzsäure, extrahiert die vereinigten salzsauren Phasen mit 50 ml Chloroform, versetzt mit 100 ml 1:1 verdünnter Ammoniaklösung und schüttelt dreimal mit 50 ml Chloroform aus, wobei das ausgefallene Produkt in Lösung geht.

Die vereinigten Chloroformphasen werden mit 50 ml Wasser ausgeschüttelt, über $Na_2SO_4$ getrocknet und im Vakuum zur Trockne eingeengt.

Den Rückstand löst man in 30 ml Isopropanol unter Erwärmen, kühlt die Lösung wieder ab und leitet Chlorwasserstoff bis zur sauren Reaktion ein. Es fällt ein schmieriges Produkt aus, das bei Aufkochen kristallisiert. Es wird abgesaugt und bei Raumtemperatur getrocknet. Die Ausbeute beträgt 6,2 g (70,4 % der Theorie), Schmelzpunkt: 127 - 150° C (unter Zersetzung).

| Analyse für $C_{20}H_{23}N_3O_4Cl_2$: | berechnet | gefunden |
|---|---|---|
| C: | 54,56 % | 54,43 % |
| H: | 5,27 % | 6,01 % |
| N: | 9,54 % | 9,03 % |
| Cl: | 16,10 % | 15,48 % |

Beispiel 9

8-Chlor-5,10-dihydro-5-[2-hydroxyäthyl)-aminocarbonyl]-11H-dibenzo[b,e][1,4]diazepin-11-on

12,3 g (0,04 Mol) 8-Chlor-5-chlorcarbonyl-5,10-dihydro-11H-dibenzo[b,e][1,4]diazepin-11-on (Schmelzpunkt: 257 - 260° C) und 4,0 g (0,04 Mol) Triäthylamin werden in 200 ml Chloroform unter Rühren und Rückfluß erhitzt und innerhalb einer halben Stunde 5,3 g (0,05 Mol) Diäthanolamin zugetropft. Man rührt das Reaktionsgemisch noch eine halbe Stunde am Rückfluß, schüttelt nach dem Abkühlen die Chloroformlösung mit 100 ml Wasser aus, trocknet kurz über Natriumsulfat und engt sie im Vakuum zur Trockne ein.

Der verbleibende Rückstand wird aus 20 ml Isopropanol umkristallisiert.

Die Ausbeute betrug 9,7 g (66,9 % der Theorie), Schmelzpunkt: 190 - 195° C (unter Zersetzung).

Analyse für $C_{18}H_{18}N_3O_4Cl$:

| | C | H | N | Cl |
|---|---|---|---|---|
| berechnet: | 57,60 % | 4,83 % | 11,18 % | 9,44 % |
| gefunden: | 58,09 % | 4,91 % | 11,37 % | 9,45 % |

Beispiel 10

8-Chlor-5,10-dihydro-5-{3-[bis-(2-hydroxyäthyl)-amino]propionyl}-11H-dibenzo[b,e][1,4]diazepin-11-on

40,2 g (0,12 Mol) 8-Chlor-5-chlorpropionyl-5,10-dihydro-11H-dibenzo[b,e][1,4]diazepin-11-on und 27,4 g (0,26 Mol) Diäthanolamin wurden in 300 ml Chloroform 6 Stunden am Rückfluß gerührt. Nach dem

Abkühlen trennt man das ausgefallene Diäthanolaminhydrochlorid ab, engt die Chloroformphase im Vakuum zur Trockne ein, verteilt den Rückstand zwischen 300 ml Chloroform und 200 ml 1:2 verdünnter HCl und trennt die salzsaure Phase ab. Nach dem Unterschichten der salzsauren Phase mit 300 ml Chloroform wird mit 1:1 verdünnter Ammoniaklösung der pH-Wert 10 eingestellt, gut durchgeschüttelt, die Chloroformphase abgetrennt und kurz über $Na_2SO_4$ getrocknet. Nach einiger Zeit fällt das Endprodukt (Rohausbeute: 28,1 g = 58 % der Theorie, Fp.: 164 - 168 °C) aus.
Es wird aus 750 ml Acetonitrol mit CFO-Kohle umkristallisiert. Das Endprodukt ist noch durch Acryloylverbindung verunreinigt.
Die Ausbeute beträgt 19,1 g (39,4 % der Theorie), Schmelzpunkt: 165 - 171 °C.

Hydrochlorid

14 g (0,035 Mol) 8-Chlor-5,10-dihydro-5-{3-[bis-(2-hydroxyäthyl)-amino]-propionyl}-11H-dibenzo[b,e]-[1,4]diazepin-11-on werden in der Siedehitze in 200 ml Isopropanol gelöst und nach dem Abkühlen mit 150 ml isopropanolischer Salzsäure bis zur sauren Reaktion versetzt. Man engt die Lösung im Vakuum zur Trockne ein, gibt dreimal je 10 ml Isopropanol zu, engt jedesmal im Vakuum zur Trockne ein, setzt nochmals 15 ml Isopropanol zu, kocht auf dem Dampfbad auf, saugt das Kristallisat nach dem Abkühlen ab und trocknet bei Raumtemperatur. Das Hydrochlorid wird aus 100 ml 96 %-igem Alkohol mit CFO-Kohle umkristallisiert. Die Ausbeute beträgt 5,3 g (34,6 % der Theorie), Schmelzpunkt: 176 - 186 °C (unter Zersetzung).

Analyse für $C_{20}H_{23}N_3O_4Cl_2$:

| | C | H | N | Cl |
|---|---|---|---|---|
| berechnet: | 54,56 % | 5,27 % | 9,54 % | 16,10 % |
| gefunden: | 54,58 % | 5,44 % | 10,22 % | 16,26 % |

Beispiel 11

8-Chlor-5,10-dihydro-5-(2-{4-[(2-furanyl)-carbonyl]-1-piperazinyl}-propionyl)-11H-dibenzo[b,e][1,4]-diazepin-11-on
12,2 g (0,04 Mol) 5-(2-Brom-propionyl)-8-chlor-5,10-dihydro-11H-dibenzo[b,e][1,4]diazepin-11-on, 17,2 g (0,04 Mol) 1-[(2-Furanyl)-carbonyl]-piperazinhydrochlorid und 16 g (0,16 Mol) Triäthylamin werden in 200 ml Dioxan 8 Stunden unter Rückfluß gerührt, nochmals 4,4 g (0,01 Mol) 1-[(2-Furanyl)-carbonyl]-piperazin-hydrochlorid
und 1,0 g (0,01 Mol) Triäthylamin zugesetzt und wiederum 8 Stunden am Rückfluß gerührt. Anschließend destilliert man das Dioxan im Vakuum ab, gibt 200 ml Chloroform und 100 ml Wasser zu, rührt gut durch, trennt die Phasen und schüttelt die Chloroformphase einmal mit 100 ml 2:1 verdünnter HCl und einmal mit 50 ml 1:2 verdünnter HCl aus.

Die vereinigten salzsauren Phasen werden mit Chloroform unterschichtet, unter Rühren und Kühlen mit konzentrierter Ammoniaklösung auf pH 9 eingestellt, die Chloroformphase abgetrennt, über $Na_2SO_4$ getrocknet und im Vakuum zur Trockne eingeengt.

Den Rückstand erwärmt man auf dem Dampfbad mit ca. 10 ml Isopropanol, läßt kristallisieren, saugt ab, wäscht mit wenig Isopropanol und trocknet bei Raumtemperatur. Das Rohprodukt wurde mit 40 ml Acetonitril ausgekocht und bei Raumtemperatur getrocknet. Die Ausbeute beträgt 7,3 g (38,1 % der Theorie), Schmelzpunkt: 238- 244 °C (unter Zersetzung).

Analyse für $C_{25}H_{23}N_4O_2Cl$:

| | C | H | N | Cl |
|---|---|---|---|---|
| berechnet: | 62,70 % | 4,84 % | 10,87 % | 7,40 % |
| gefunden: | 62,10 % | 5,15 % | 11,45 % | 7,40 % |

Hydrochlorid

22,7 g (0,047 Mol) 8-Chlor-5,10-dihydro-5-(2-{4-[(2-furanyl)-carbonyl]-1-piperazinyl}-propionyl)-11H-dibenzo[b,e][1,4]diazepin-11-on werden in 200 ml Isopropanol suspendiert, mit 200 ml isopropanolischer Salzsäure (ca. 19 %-ig) versetzt und eine halbe Stunde auf dem Wasserbad erwärmt. Es verbleibt ein Rückstand, den man absaugt. Die Lösung wird im Vakuum zur Trockne eingeengt. Den schmierigen Rückstand versetzt man mit 20 ml Isopropanol, engt im Vakuum zur Trockne ein und nimmt nochmals mit 20 ml absolutem Äthanol auf und engt wiederum im Vakuum zur Trockne ein. Den Rückstand kristallisiert man aus 175 ml 60 %-igem Isopropanol mit CFO-Kohle um und trocknet nochmals wie oben angegeben. Die Ausbeute beträgt 6,2 g (25 % der Theorie), Schmelzpunkt: 212°C (unter Zersetzung).

Analyse für das Semihydrat $C_{25}H_{25}N_4O_{4,5}Cl_2$:

| | C | H | N | Cl | 0,5 Mol $H_2O$ |
|---|---|---|---|---|---|
| berechnet: | 57,26 % | 4,81 % | 10,68 % | 13,52 % | 3,40 % |
| gefunden: | 57,23 % | 5,02 % | 10,73 % | 12,38 % | 2,50 % |

Versetzt man die Mutterlauge der Umkristallisation mit 500 ml Isopropanol, kristallisiert nochmals Hydrochlorid aus, das abgesaugt und wie oben getrocknet wird.

Die Ausbeute der Nachfällung beträgt 3,0 g (12,1 % der Theorie), Schmelzpunkt: 224 - 231°C (unter Zersetzung).

Analyse für das Semihydrat $C_{25}H_{25}N_4O_{4,5}Cl_2$:

| | C | H | N | Cl |
|---|---|---|---|---|
| berechnet: | 57,26 % | 4,81 % | 10,68 % | 13,52 % |
| gefunden: | 57,51 % | 4,74 % | 10,49 % | 13,41 % |

Beispiel 12

8-Chlor-5,10-dihydro-5-({4-[(2-furanyl)-carbonyl]-1-piperazinyl}-acetyl)-10-methyl-11H-dibenzo[b,e][1,4]-diazepin-11-on

6,7 g (0,02 Mol) 8-Chlor-5-chloracetyl-5,10-dihydro-10-methyl-11H-dibenzo[b,e][1,4]diazepin-11-on, 8,8 g (0,04 Mol) 1-[(2-Furanyl)-carbonyl]-piperazinhydrochlorid und 6,0 g (0,06 Mol) Triäthylamin werden in 70 ml Chloroform 7 Stunden am Rückfluß gerührt. Zur abgekühlten Reaktionslösung gibt man 100 ml Wasser, rührt gut durch, trennt die Phasen und extrahiert die Chloroformphase mit 100 ml 2:1 verdünnter Salzsäure. Zur salzsauren Phase werden 100 ml Chloroform und 140 ml 1:1 verdünnte Ammoniaklösung gegeben, gut durchgerührt, die abgetrennte Chloroformphase über $Na_2SO_4$ getrocknet und im Vakuum zur Trockne eingeengt. Den Rückstand kocht man mit 10 ml Isopropanol auf, saugt ab, trocknet bei Raumtemperatur und kristallisiert aus absolutem Alkohol mit CFO-Kohle um.

Die Ausbeute beträgt 5,4 g (56,01 % der Theorie), Schmelzpunkt: 189 - 194°C (unter Zersetzung).

Analyse für $C_{25}H_{23}N_4O_4Cl$:

| | C | H | N | Cl |
|---|---|---|---|---|
| berechnet: | 62,70 % | 4,84 % | 11,70 % | 7,40 % |
| gefunden: | 62,33 % | 4,89 % | 11,43 % | 7,61 % |

Hydrochlorid

14,4 g (0,03 Mol) 8-Chlor-5,10-dihydro-5-({4-[(2-furanyl)-carbonyl]-1-piperazinyl}-acetyl)-10-methyl-11H-dibenzo[b,e][1,4]diazepin-11-on werden in 80 ml Isopropanol suspendiert und mit 100 ml isopropanolischer Salzsäure (ca. 19 %-ig) versetzt. Anschließend erwärmt man leicht, wobei sich die gesamte freie Base löst. Die Lösung wird im Vakuum zur Trockne eingeengt und mit Alkohol aufgekocht, wobei eine schmierige feste Masse kristallisiert. Der Rückstand wird mit Isopropanol wieder in Lösung gebracht und mit 500 ml Äther das Hydrochlorid ausgefällt. Man saugt das Kristallisat ab, trocknet im Exsikkator über NaOH (Wasserstrahlvakuum) und 8 Stunden bei 2 Torr über $P_2O_5$. Die Ausbeute beträgt 11,4 g (72,3 % der Theorie), Schmelzpunkt: 100° C / 160 - 169° C (unter Zersetzung).

Analyse für das Hydrat $C_{25}H_{25}N_4O_{4,5}Cl_2$:

| | C | H | N | Cl | 0,5 Mol $H_2O$ |
|---|---|---|---|---|---|
| berechnet: | 57,26 % | 4,81 % | 10,68 % | 13,73 % | 1,72 % |
| gefunden: | 57,43 % | 5,04 % | 10,48 % | 13,53 % | 2,40 % |

Analog den Beispielen 1 bis 12 wurden die in der folgenden Tabelle aufgeführten Verbindungen hergestellt.

| Beispiel | $R^1$ | $R^2$ | $R^3$ | $R^4$ | A | Schmelzpunkt | Ausbeute % |
|---|---|---|---|---|---|---|---|
| 13 | Cl | H | $-C_2H_4OH$ | $-CH_3$ | $-CH_2-$ | 161 - 162,5 °C (Butylacetat)<br>Hydrochlorid:<br>244,5 - 246 °C (unter Zersetzung) | 59,7 |
| 14 | Cl | H | $-H$ | $-C_2H_4OH$ | $-CH_2-$ | 134 - 139 °C (unter Zersetzung) (Isopropanol)<br>Hydrochlorid:<br>252,5 - 255 °C (unter Zersetzung) | 49,6 |
| 15 | Cl | H | $-CH_3$ | $-CH_2CO-$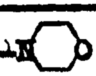 | $-CH_2-$ | 120 °C (unter Zersetzung) (Ethansäureethylester)<br>Hydrochlorid:<br>204 °C (unter Zersetzung) | 54,2 |

| Beispiel | $R^1$ | $R^2$ | $R^3$ | $R^4$ | A | Schmelzpunkt | Ausbeute % |
|---|---|---|---|---|---|---|---|
| 16 | Cl | H | H | $-CH_2CH_2NH-\underset{\underset{O}{\|}}{C}-CH_3$ | $-CH_2-$ | Hydrochlorid: 223 - 226 °C (unter Zersetzung) (Isopropanol) | 58,9 |
| 17 | Cl | H | $-C_2H_4OH$ | $-C_2H_4OH$ | $-CH(CH_3)-$ | 167 - 170 °C (Isopropanol) | 7,4 |

| Beispiel | $R^1$ | $R^2$ | $R^3$ | $R^4$ | A | Schmelzpunkt | Ausbeute % |
|---|---|---|---|---|---|---|---|
| 18 | H | H | $-CH_3$ | 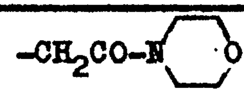 | $-CH_2-$ | 172,5 - 177 °C (Isopropanol) Hydrochlorid: 190 °C (unter Zersetzung) | 57,6 |
| 19 | H | $-CH_3$ | 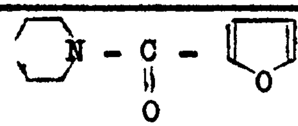 | | $-CH_2-$ | (161 °C) 166 - 170 °C (Isopropanol) Hydrochlorid: 160 °C (unter Zersetzung) | 65,2 |

Beispiel 20

5,10-Dihydro-5-[bis-(2-hydroxyethyl)-aminoacetyl]-11H-dibenzo[b,e][1,4]diazepin-11-on.HCl

46 g (0,16 Mol) 5-Chloracetyl-5,10-dihydro-11H-dibenzo[b,e][1,4]-diazepin-11-on, 200 ml Dimethylformamid und 33,76 g (0,32 Mol) Diethanolamin werden 8 Stunden bei Raumtemperatur gerührt und 12 Stunden bei dieser Temperatur stehengelassen. Danach versetzt man das Reaktionsgemisch mit 1 g CFO-Kohle, rührt 1 Stunde bei Raumtemperatur, saugt die Kohle ab und engt die Lösung im Vakuum zur Trockne ein. Der flüssige Rückstand wird mit 200 ml Chloroform versetzt, mit 20 ml Wasser ausgeschüttelt und wiederum eingeengt. Man gibt dann 200 ml Aceton zu und versetzt mit frisch hergestellter acetonischer Salzsäure oder konzentrierter Salzsäure (ca. 15 ml) bis zur sauren Reaktion. Es setzt sich zuerst eine schmierige Masse ab, die langsam kristallisiert (Ausbeute: 54 g ≙ 86,1 % der Theorie). Das Rohprodukt wird abgesaugt, mit Aceton gewaschen, in der 12 bis 13-fachen Menge Methanol gelöst, eine halbe Stunde mit 5 g CFO-Kohle erhitzt, klargesaugt, auf etwa 1/3 eingeengt und mit 200 ml Aceton versetzt. Das Kristallisat löst man nochmals in der 15-fachen Menge Methanol, erhitzt wiederum mit 2,5 g CFO-Kohle, saugt ab, engt auf 1/3 ein und saugt das Kristallisat ab. Das Produkt wird noch mit der 5-fachen Menge Aceton ausgekocht. Die Ausbeute beträgt 43,5 g ≙ 69,4 % der Theorie.
Schmelzpunkt: 208 °C (unter Zersetzung).

**Ansprüche**

1. Verfahren zur Herstellung von neuen in 5-Stellung substituierten 5,10-Dihydro-11H-dibenzo(b,e)(1,4)-diazepin-11-onen der allgemeinen Formel

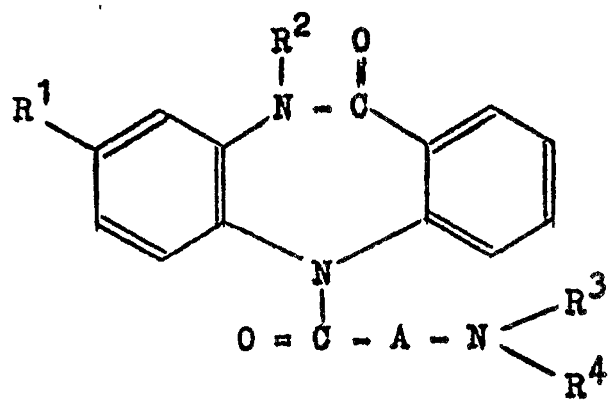

(I)

und ihren physiologisch verträglichen Salzen mit anorganischen oder organischen Säuren, worin $R^1$ Wasserstoff oder Chlor, $R^2$ Wasserstoff oder Methyl, $R^3$ Wasserstoff, Methyl oder 2-Hydroxiäthyl, $R^4$ 2-Hydroxiäthyl, N-Morpholylcarbonylmethyl oder 2-Acetylaminoäthyl oder $R^3$ und $R^4$ zusammen mit dem N-Atom, an das sie gebunden sind, einen Piperazinrest, der an der Iminogruppe durch einen (2-Furanyl) -carbonyl- oder einen 3,4,5-Trimethoxibenzoylrest
substituiert ist, und A eine Gruppe der Formel $-CH_2-$, $-CH_2-CH_2-$oder $-CH(CH_3)-$ oder eine direkte Bindung bedeuten, dadurch gekennzeichnet, daß man entweder

a) ein 5,10-Dihydro-5-halogenacyl-11H-dibenzo(b,e)(1,4)-diazepin-11-on der allgemeinen Formel

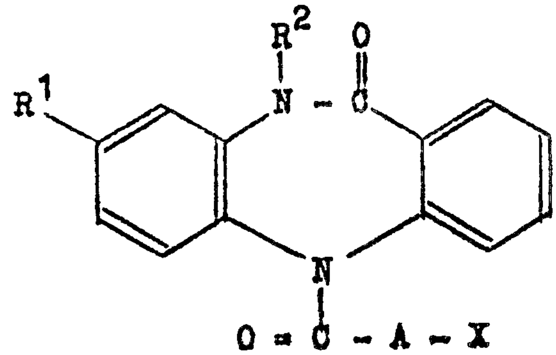

(II)

worin $R^1$, $R^2$ und A die obengenannte Bedeutung besitzen und X ein Halogenatom bedeutet, mit einem Amin der allgmeinen Formel

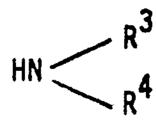

(III)

in der $R^3$ und $R^4$ die obengenannte Bedeutung besitzen, oder einem Hydrochlorid eines Amins der allgemeinen Formel

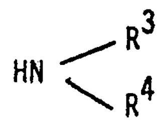

(III)

umsetzt, oder

b)eine Verbindung der allgemeinen Formel

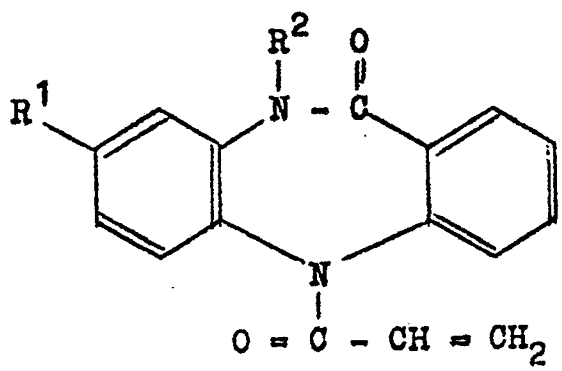

(VI)

worin $R^1$ und $R^2$ die obengenannte Bedeutung besitzen, mit einem Amin der allgemeinen Formel

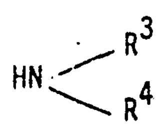

(III)

worin $R^3$ und $R^4$ die obengenannte Bedeutung besitzen, umsetzt, oder

c)ein 5,10-Dihydro-11H-dibenzo(b,e)(1,4)diazepin-11-on der allgemeinen Formel

(IV)

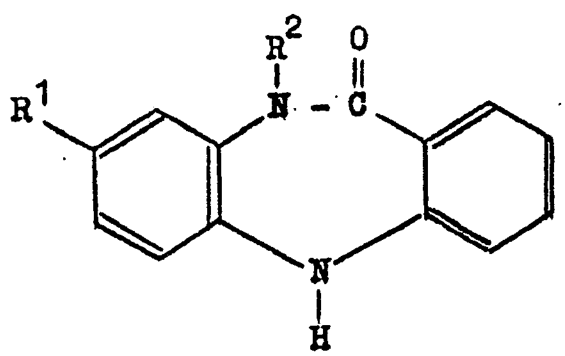

worin $R^1$ und $R^2$ die obengenannte Bedeutung besitzen, mit einer Verbindung der allgemeinen Formel

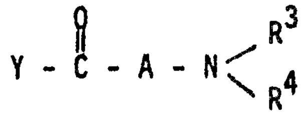

(VII)

in der $R^3$, $R^4$ und A die obengenannte Bedeutung besitzen und Y die Hydroxylgruppe oder ein Halogenatom bzw., wenn A eine direkte Bindung ist, nur ein Halogenatom bedeutet, oder deren Salze umsetzt.

**2.** Verfahren nach Anspruch 1, dadurch gekennzeichnet, daß die Umsetzungen in einem inerten organischen Lösungsmittel durchgeführt werden.

**3.** Verfahren nach den Ansprüchen 1a und 2, dadurch gekennzeichnet, daß die Umsetzung der Verbindungen der allgemeinen Formel II mit den Verbindungen der allgemeinen Formel III oder deren Hydrochloriden bei Temperaturen zwischen Raumtemperatur und 150° C, vorzugsweise zwischen 50 und 120° C, durchgeführt wird.

**4.** Verfahren nach den Ansprüchen 1a, 2 und 3, dadurch gekennzeichnet, daß als indifferente organische Lösungsmittel bei der Umsetzung der Verbindungen der allgemeinen Formel II mit den Verbindungen der allgemeinen Formel III oder deren Hydrochloriden Alkohole wie Äthanol, n-Propanol, Isopropanol, Äther wie Dioxan oder Tetrahydrofuran, Ketone wie Aceton oder Cyclohexanon, aromatische Kohlenwasserstoffe wie Benzen oder Toluen oder halogenierte aliphatische oder aromatische Kohlenwasserstoffe wie Chloroform, 1,2-Dichloräthan oder Chlorbenzen verwendet werden.

**5.** Verfahren nach den Ansprüchen 1a und 2 bis 4, dadurch gekennzeichnet, daß als Lösungsmittel ein Überschuß des eingesetzten Amins der allgemeinen Formel III verwendet wird.

**6.** Verfahren nach den Ansprüchen 1a und 2 bis 5, dadurch gekennzeichnet, daß die Umsetzung der Verbindungen der allgemeinen Formel II mit den Verbindungen der allgemeinen Formel III in Gegenwart eines Säureacceptors durchgeführt wird.

**7.** Verfahren nach den Ansprüchen 1a und 2 bis 6, dadurch gekennzeichnet, daß als Säureacceptor Alkalicarbonate oder Alkalihydrogencarbonate verwendet werden.

**8.** Verfahren nach den Ansprüchen 1a und 2 bis 6, dadurch gekennzeichnet, daß als Säureacceptoren tertiäre Amine wie Triäthylamin, Pyridin oder N,N-Dimethylanilin verwendet werden.

**9.** Verfahren nach den Ansprüchen 1b und 2, dadurch gekennzeichnet, daß bei der Umsetzung einer Verbindung der allgemeinen Formel VI mit einer Verbindung der allgemeinen Formel III als indifferente organische Lösungsmittel Alkohole wie Äthanol, n-Propanol oder Isopropanol, Ketone wie Aceton oder Cyclohexanon, höhersiedende Äther wie Dioxan oder Tetrahydrofuran oder aromatische Kohlenwasserstoffe wie Benzen oder Toluen eingesetzt werden.

**10.** Verfahren nach den Ansprüchen 1b, 2 und 9, dadurch gekennzeichnet, daß die Umsetzung der Verbindungen der allgemeinen Formel VI mit den Aminen der allgemeinen Formel III unter Erwärmen, vorzugsweise bis zur Siedetemperatur des Reaktionsgemisches, erfolgt.

**11.** Verfahren nach den Ansprüchen 1c und 2, dadurch gekennzeichnet, daß als inerte organische Lösungsmittel halogenierte Kohlenwasserstoffe wie Chloroform oder Dichlormethan eingesetzt werden.

**12.** Verfahren nach den Ansprüchen 1c, 2 und 11, dadurch gekennzeichnet, daß die Umsetzung unter Erwärmen bis zur Siedetemperatur durchgeführt wird.

**13.** Verfahren nach den Ansprüchen 1c, 2, 11 und 12, dadurch gekennzeichnet, daß, wenn Y in der allgemeinen Formel VII eine Hydroxylgruppe bedeutet, die Umsetzung in Gegenwart von 1-Methyl-2-halogenpyridiniumjodid und Triäthylamin durchgeführt wird.

14. Neue in 5-Stellung substituierte 5,10-Dihydro-11H-dibenzo [b,e][1,4]diazepin-11-one der allgemeinen Formel

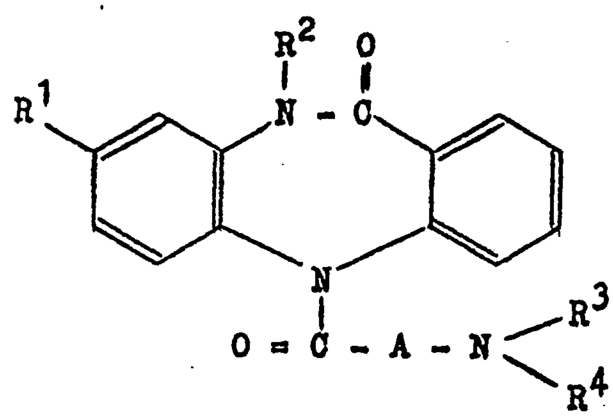

(I)

und ihre physiologisch verträglichen Salze mit anorganischen oder organischen Säuren, worin $R^1$ Wasserstoff oder Chlor, $R^2$ Wasserstoff oder Methyl, $R^3$ Wasserstoff, Methyl oder 2-Hydroxyäthyl, $R^4$ 2-Hydroxyäthyl, N-Morpholylcarbonylmethyl oder 2-Acetylaminoäthyl oder $R^3$ und $R^4$ zusammen mit dem N-Atom, an das sie gebunden sind, einen Piperazinrest, der an der Iminogruppe durch einen (2-Furanyl)-carbonyl-, oder einen 3,4,5,-Trimethoxybenzoylrest substituiert ist, und A eine Gruppe der Formel $-CH_2-$, $-CH_2-CH_2-$ oder $-CH(CH_3)-$ oder eine direkte Bindung bedeuten.

## Claims

1. Process for the production of new 5-substituted 5, 10-dihydro-11H-dibenzo(b,e)(1,4)diazepin-11-ones of the general formula

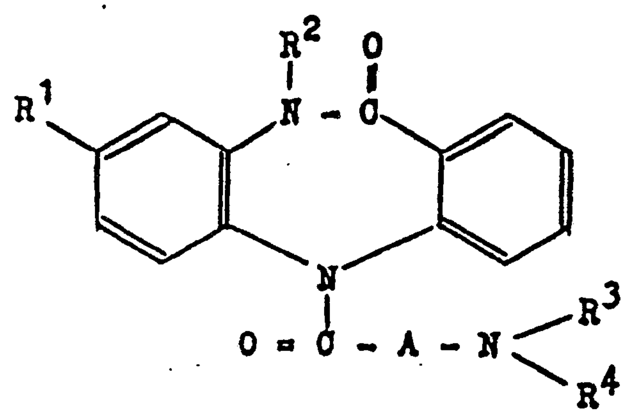

(I)

and their physiologically tolerable salts with inorganic or organic acids, in which $R^1$ signifies hydrogen or chlorine, $R^2$ hydrogen or methyl, $R^3$ hydrogen, methyl or 2-hydroxyethyl, $R^4$ 2-hydroxyethyl, N-morpholylcarbonylmethyl or 2-acetylaminoethyl, or $R^3$ and $R^4$ together with the N atom to which they are linked signify a piperazine group which is substituted at the imino group by a (2-furanyl)-carbonyl or a 3,4,5-trimethoxybenzoyl group, and A a group of the formula $-CH_2-$, $-CH_2-CH_2-$ or $-CH(CH_3)-$ or a direct bond, characterized in that either

(a) a 5,10-dihydro-5-halogenoacyl-11H-dibenzo(b,e)(1,4)diazepin-11-one of the general formula

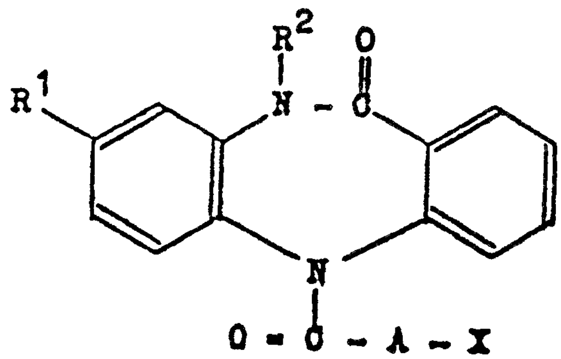

(II)

in which $R^1$, $R^2$ and A have the above significance and X signifies a halogen atom is reacted with an amine of the general formula

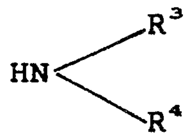

(III)

in which $R^3$ and $R^4$ have the above significance, or a hydrochloride of an amine of the general formula

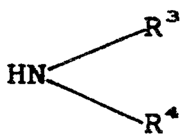

(III)

or (b) a compound of the general formula

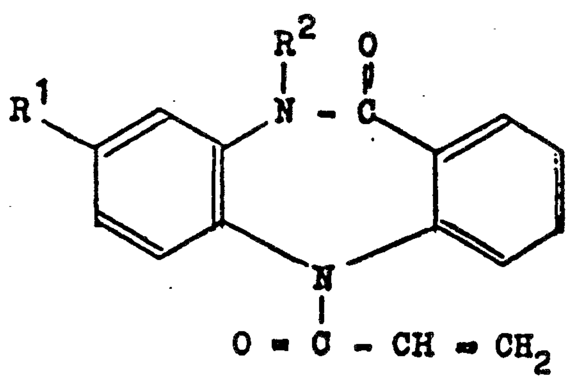

(VI)

in which $R^1$ and $R^2$ have the above significance is reacted with an amine of the general formula

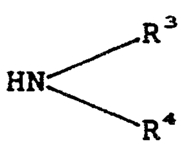

(III)

in which $R^3$ and $R^4$ have the above significance, or
(c) a 5,10-dihydro-11H-dibenzo(b,e)(1,4)diazepin-11-one of the general formula

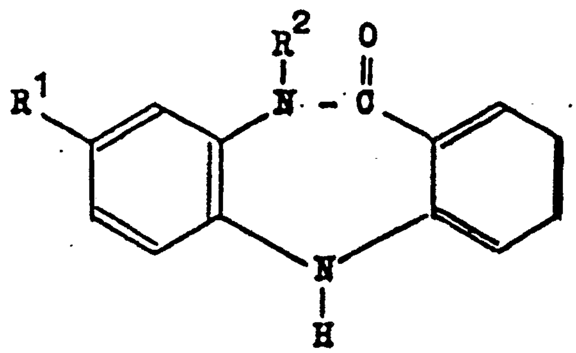

(IV)

in which $R^1$ and $R^2$ have the above significance is reacted with a compound of the general formula

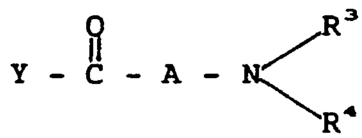

(VII)

in which $R^3$, $R^4$ and A have the above significance and Y is the hydroxyl group or a halogen atom or, if A is a direct bond, only a halogen atom, or its salts.

2. Process according to Claim 1, characterized in that the reactions are carried out in an inert organic solvent.

3. Process according to Claims 1a and 2, characterized in that the reaction of the compounds of general formula II with the compounds of general formula III or their hydrochlorides is carried out at temperatures between room temperature and 150 °C, preferably between 50 and 120 °C.

4. Process according to Claims 1a, 2 and 3, characterized in that in the reactions of compounds of general formula II with compounds of general formula III or their hydrochlorides, alcohols such as ethanol, n-propanol or isopropanol, ethers such as dioxane or tetrahydrofuran, ketones such as acetone or cyclohexanone, aromatic hydrocarbons such as benzene or toluene or halogenated aliphatic or aromatic hydrocarbons such as chloroform, 1,2-dichloroethane or chlorobenzene are used as inert organic solvents.

5. Process according to Claims 1a and 2 to 4, characterized in that an excess of the amine used of general formula III is used as solvent.

6. Process according to Claims 1a and 2 to 5, characterized in that the reaction of compounds of general formula II with the compounds of general formula III is carried out in the presence of an acid acceptor.

7. Process according to Claims 1a and 2 to 6, characterized in that alkali carbonates or alkali hydrogen carbonates are used as acid acceptor.

8. Process according to Claims 1a and 2 to 6, characterized in that tertiary amines such as triethylamine, pyridine or N,N-dimethylaniline are used as acid acceptors.

9. Process according to Claims 1b and 2, characterized in that in the reaction of a compound of general formula VI with a compound of general formula III, alcohols such as ethanol, n-propanol or isopropanol, ketones such as acetone or cyclohexanone, higher-boiling ethers such as dioxane or tetrahydrofuran, or aromatic hydrocarbons such as benzene or toluene are used as inert organic solvents.

10. Process according to Claims 1b, 2 and 9, characterized in that the reaction of the compounds of general formula VI with the amines of general formula III is carried out with heating, preferably to the boiling point of the reaction mixture.

**11.** Process according to Claims 1c and 2, characterized in that halogenated hydrocarbons such as chloroform or dichloromethane are used as inert organic solvents.

**12.** Process according to Claims 1c, 2 and 11, characterized in that the reaction is carried out with heating to the boiling point.

**13.** Process according to Claims 1c, 2, 11, and 12, characterized in that if Y in general formula VII signifies a hydroxyl group, the reaction is carried out in the presence of 1-methyl-2-halogeno-pyridinium iodide and triethylamine.

**14.** New 5-substituted 5,10-dihydro-11H-dibenzo [b,e][1,4]diazepin-11-ones of the general formula

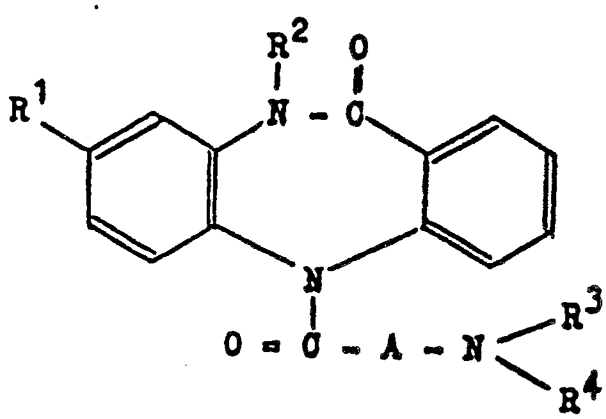

(I)

and their physiologically tolerable salts with inorganic or organic acids, in which $R^1$ signifies hydrogen or chlorine, $R^2$ hydrogen or methyl, $R^3$ hydrogen, methyl or 2-hydroxyethyl, $R^4$ 2-hydroxyethyl, N-morpholylcarbonylmethyl or 2-acetylaminoethyl, or $R^3$ and $R^4$ together with the N atom to which they are linked signify a piperazine group which is substituted at the imino group by a (2-furanyl)-carbonyl or a 3,4,5-trimethoxybenzoyl group, and A a group of the formula $-CH_2-$, $-CH_2-CH_2-$ or $-CH(CH_3)-$ or a direct bond.

## Revendications

**1.** Procédé pour la préparation de nouvelles 5,10-dihydro-11H-dibenzo(b,e)(1,4)diazépine-11-ones substituées en position 5, de formule générale

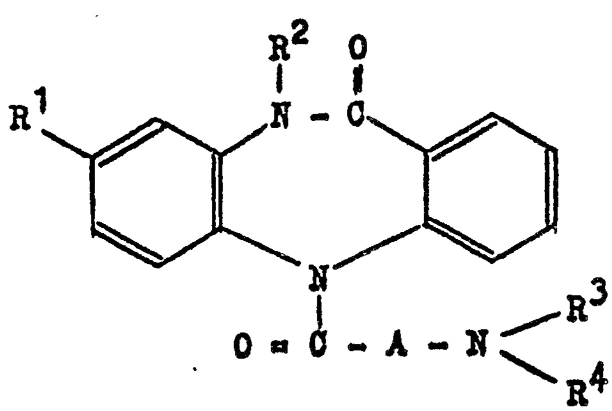

(I)

et de leurs sels physiologiquement supportables avec des acides non organiques ou organiques, où $R^1$ représente l'hydrogène ou le chlore, $R^2$ représente l'hydrogène ou méthyle, $R^3$ représente l'hydrogène, méthyle ou 2-hydroxyéthyle, $R^4$ représente 2-hydroxyéthyle, N-morpholylcarbonylméthyle ou 2-acétylaminoéthyle ou $R^3$ et $R^4$, ensemble avec l'atome de N auquel ils sont liés, représentent un radical

pipérazine, qui est substitué sur le groupe imino par un radical (2-furanyl)-carbonyle ou un radical 3,4,5-triméthoxybenzoyle, et A représente un groupe de formule $-CH_2-$, $-CH_2-CH_2-$ ou $-CH(CH_3)-$ ou une liaison directe, caractérisé en ce que soit :

a) on fait réagir une 5,10-dihydro-5-halogènoacyl-11H-dibenzo(b,e)(1,4)-diazépine-11-one de formule générale

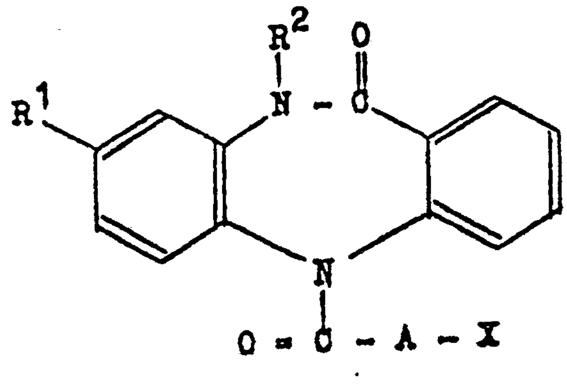

(II)

dans laquelle $R^1$, $R^2$ et A possèdent la signification susmentionnées et X représente un atome d'halogène, avec une amine de formule générale

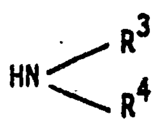

(III)

dans laquelle $R^3$ et $R^4$ possèdent la signification susmentionnée, ou avec un chlorhydrate d'une amine de formule générale

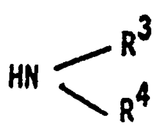

(III)

soit

b) ont fait réagir un composé de formule générale

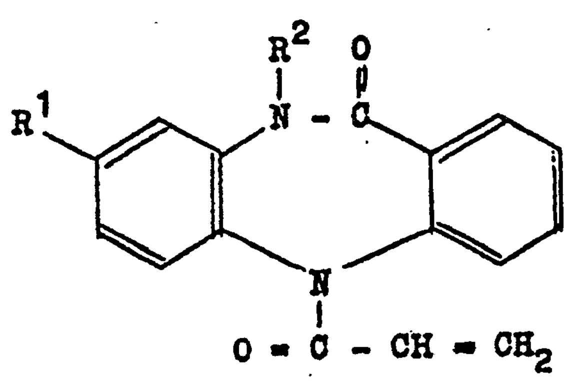

(VI)

dans laquelle $R^1$ et $R^2$ possèdent la significaton susmentionnée, avec une amine de formule générale

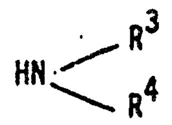

(III)

dans laquelle $R^3$ et $R^4$ possèdent la signification susmentionnée, soit

c) on fait réagir une 5,10-dihydro-11H-dibenzo(b,e)(1,4)diazépine-11-one de formule générale

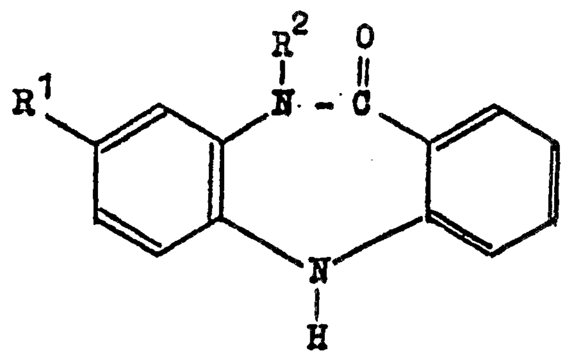

(IV)

dans laquelle $R^1$ et $R^2$ possèdent la signification susmentionnée, avec un composé de formule générale

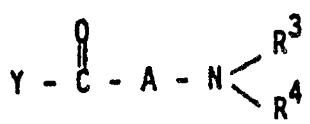

(VII)

dans laquelle $R^3$, $R^4$ et A possèdent la signification susmentionnée et Y est le groupe hydroxyle ou un atome d'halogène respectivement, lorsque A est une liaison directe, seulement un atome d'halogène, ou ses sels.

**2.** Procédé selon la revendication 1, caractérisé en ce que les réactions sont effectuées dans un solvant organique inerte.

**3.** Procédé selon les revendications la et 2, caractérisé en ce que la réaction des composés de formule générale II avec les composés de formule générale III ou leurs chlorhydrates est effectuée à des températures entre la température ambiante et 150° C, de préférence entre 50 et 120° C.

**4.** Procédé selon les revendications 1a, 2 et 3, caractérisé en ce que l'on utilise, en tant que solvants organiques indifférents, lors de la réaction des composés de formule générale II avec les composés de formule générale III ou leurs chlorhydrates, des alcools tels que l'éthanol, le n-propanol, l'isopropanol, des éthers tels que le dioxane ou le tétrahydrofurane, des cétones telles que l'acétone ou la cyclohexanone, des hydrocarbures aromatiques tels que le benzène ou le toluène ou des hydrocarbures halogènés aliphatiques ou aromatiques tels que le chloroforme, le 1,2-dichloroéthane ou le chlorobenzène.

**5.** Procédé selon les revendications 1a et 2 à 4, caractérisé en ce qu'on utilise, comme solvant un excès de l'amine de formule générale III mise en oeuvre.

**6.** Procédé selon les revendications 1a et 2 à 5, caractérisé en ce que la réaction des composés de formule générale II avec les composés de formule générale III est effectuée en présence d'un accepteur d'acides.

**7.** Procédé selon les revendications 1a et 2 à 6, caractérisé en ce qu'on utilise, comme accepteurs d'acides, des carbonates alcalins ou des bicarbonates alcalins.

**8.** Procédé selon les revendications 1a et 2 à 6, caractérisé en ce qu on utilise, comme accepteurs d'acides, des amines tertiaires comme la triéthylamine, la pyridine ou la N,N-diméthylaniline.

**9.** Procédé selon les revendications 1b et 2, caractérisé en ce que, lors de la réaction d'un composé de formule générale VI avec un composé de formule générale III, on met en oeuvre, comme solvants organiques indifférents, des alcools tels que l'éthanol, le n-propanol ou l'isopropanol, des cétones comme l'acétone ou la cyclohexanone, des éthers à point d'ébullition relativement élevé comme le dioxane ou le tétrahydrofurane ou des hydrocarbures aromatiques tels que le benzène ou le toluène.

**10.** Procédé selon les revendications 1b, 2 et 9, caractérisé en ce que la réaction des composés de formule générale VI avec les amines de formule générale III a lieu en chauffant, de préférence jusqu'à la température d'ébullition du mélange réactionnel.

**11.** Procédé selon les revendications 1c et 2, caractérisé en ce que l'on met en oeuvre comme solvants organiques inertes, des hydrocarbures halogènés comme le chloroforme ou le dichlorométhane.

**12.** Procédé selon les revendications 1c, 2 et 11, caractérisé en ce que la réaction est effectuée en chauffant jusqu'à la température d'ébullition.

**13.** Procédé selon les revendications 1c, 2, 11 et 12, caractérisé en ce que, lorsque Y dans la formule générale VII représente un groupe hydroxyle, la réaction est effectuée en présence d'iodure de 1-méthyl-2-halogèno-pyridinium et de triéthylamine.

**14.** Nouvelles 5,10-dihydro-11H-dibenzo[b,e][1,4]diazépine-11-ones, substituées en position 5, de formule générale

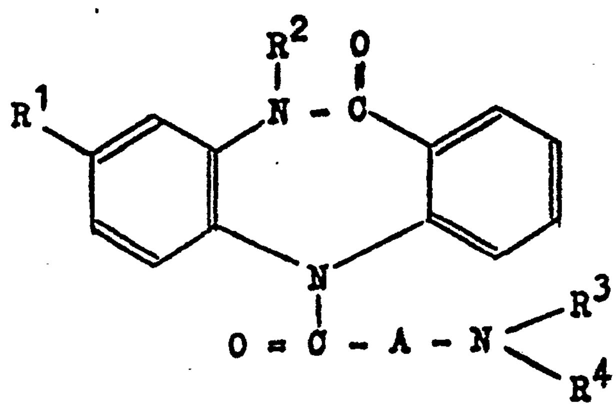

(I)

et leurs sels physiologiquement supportables aves des acides non organiques ou organiques, où $R^1$ représente l'hydrogène ou le chlore, $R^2$ représente l'hydrogène ou méthyle, $R^3$ représente l'hydrogène, méthyle ou 2-hydroxyéthyle, $R^4$ représente 2-hydroxyéthyle, N-morpholylcarbonylméthyle ou 2-acétylaminoéthyle ou $R^3$ et $R^4$, ensemble avec l'atome de N auquel ils sont liés, représentent un radical pipérazine, qui est substitué par un radical (2-furanyl)-carbonyle ou un radical 3,4,5-triméthoxybenzoyle sur le groupe imino, et A représente un groupe de formule $-CH_2-$, $-CH_2-CH_2-$ ou $-CH(CH_3)-$ ou une liaison directe.